# EUROPEAN PATENT SPECIFICATION

(11) **EP 2 672 902 B1**
(45) Date of publication and mention of the grant of the patent: **25.11.2015**
(21) Application number: 12744898.3
(22) Date of filing: 10.02.2012
(51) Int. Cl.: A61B 17/16

(54) **DRILL BIT**
BOHRMEISSEL
TRÉPAN

(30) Priority: 11.02.2011 AU 2011900459
(43) Date of publication of application: 18.12.2013
(73) Proprietor: CPL Holdings Pty Ltd, Blackalls Park, NSW 2283 (AU)
(72) Inventor: ELLIS, Liam Patrick, Blackalls Park, NSW 2283 (AU)
(74) Representative: Henriksson, Mikael
(86) International application number: PCT/AU2012/000130
(87) International publication number: WO 2012/106773

(56) References cited:
- EP-B1- 0 790 092
- WO-A1-2004/014241
- US-A- 5 273 380
- US-A- 5 452 971
- US-A- 5 584 617
- US-A- 5 759 185
- US-A1- 2009 216 235
- US-B1- 6 652 203
- NATALI ET AL.: 'Orthopaedic Bone Drills - Can They Be Improved? Temperature Changes Near the Drilling Face' THE JOURNAL OF BONE & JOINT SURGERY vol. 78, no. 3, May 1996, pages 357 - 362

## Description

### Technical Field

The present invention relates to the field of drill bits and in particular relates to, but is not limited to an orthopaedic drill bit.

### Background of the Invention

Drill bits are traditionally formed from a rod/shaft of high strength metallic material by grinding two or more helical gulleys, known as flutes, into the side wall of the rod extending from the operative front end of the rod towards the rear end, leaving a cylindrical shank at the rear end of the rod. The flutes are separated by lands that define the full diameter of the rod. To reduce the drag that would otherwise be experienced between the lands and the wall of the hole being drilled, the trailing region of each of the lands is ground, providing a slightly reduced diameter over this portion of the drill bit known as a relief. This leaves only a leading portion, known as a margin of the land, defining the full diameter of the drill. The leading edge of the margin defines a sharp secondary cutting edge with the trailing side wall of the adjacent flute, which is known as a cutting lip. During drilling operations, only the margin portion of the land engages the wall of the hole in the material being drilled, thereby reducing drag acting on the drill bit and, accordingly, reducing the likelihood of the drill bit binding.

The cutting end part of the drill bit is formed by grinding the end region of the rod to provide a generally conical end part, known as a point, with end or tip faces extending from each land towards either a chiselled edge, for designs with two flutes, or a sharp point tip for designs with three or more flutes. A primary cutting edge is defined by the junction between the leading edge of each of the tip faces and the adjacent trailing side wall of the adjacent flute. It is these primary cutting edges that cut material being drilled at the end of the drill hole. The shavings of swarf cut from the material pass along the flutes towards the rear of the drill bit, thereby creating room for more material to be cut or shaved and passed into and along the flutes for ejection from the rear end of the flutes.

In orthopaedic applications, typical orthopaedic drill bits can result in potential damage to soft tissue such as tendons, ligaments, adjacent tissue and other vital organs due to the cutting action of the primary and secondary cutting edges. Typical orthopaedic drill bits also lack some precision and can provide a relatively unsmooth cutting action. Flute designs in traditional drill bits also tend to engage soft tissue, resulting in the tissue being wrapped around the drill bit, leading to considerable tissue trauma. This can lead to increased trauma to the patient and, possibly, in the case of arterial damage, can lead to death.

In orthopaedic applications, most drilling procedures require the drilling of the bone through the center or hollow part of the bone known as the medullary canal. Drilling to fixate a fracture requires drilling from one side of cortex to the other. These cortices are known as the near cortex and far cortex. Beyond the far cortex lies soft tissues such as muscles, veins and arteries.

Also in some cases bone structures being drilled into generally comprise a hard, dense, thin external layer of compact or cortical bone and an inner layer of lighter, spongy or cancellous bone. The hardness and density of the cortical bone results in it being significantly tougher to drill through than the cancellous bone.

With typical orthopaedic drill bits, it is difficult to judge when the cutting end part is about to break through the cortical bone. This breakthrough occurs almost immediately after the drill bit has progressed through the bone to an extent where the rear end of the primary cutting edge (at the full diameter of the drill bit) first engages the bone surface, providing a hole in the bone surface that is the full diameter of the drill bit.

Once the drill bit breaks through the near cortex it travels through the hollow part of the bone into the far cortex where it breaks through into soft tissue. The soft tissue provides little or no resistance and the axial load applied to the drill bit by the operator, advancing the drill bit, can result in the breakthrough being sudden, with the drill bit rapidly overshooting deep into the muscles, veins and arteries beyond the required hole depth, potentially resulting in significant increased trauma and in some cases, where arterial damage may be caused, death. A known drill, on which the preamble of claim 1 is based, is disclosed in US-A-2009216235.

### Object of the Invention

It is an object of the present invention to substantially overcome or at least ameliorate at least one of the above disadvantages, or at least to provide a useful alternative to present drill bits.

### Summary of the Invention

In a first aspect, the present invention provides a drill bit having a central axis and comprising:
a tapered cutting end part terminating in a drill tip at one end of said drill bit;
a shank extending from an opposing end of said drill bit;
a body extending between said cutting end part and said shank;
a plurality of flutes formed in said drill bit and generally helically extending along said body into said cutting end part to adjacent said drill tip, each said flute having a flute leading side wall and a flute trailing side wall;
a land defined on said body between each of said flutes and extending to said cutting end part, each said land defining a secondary edge with said flute trailing side wall of the leading adjacent said flute, each said secondary edge helically extending to said cutting end part at a secondary edge helix angle;
a plurality of tip faces defined on said cutting end part and each extending from one of said lands to said drill tip, said tip faces being separated by said flutes up to adjacent said drill tip, each said tip face defining a primary cutting edge with said flute trailing side wall of the leading adjacent said flute;
wherein each said primary cutting edge extends from one of said secondary edges in a variable conic helix type manner with a primary cutting edge helix angle that decreases from substantially equal to said secondary edge helix angle at said secondary edge toward zero degrees as it approaches said drill tip.

In the context of the present specification, a variable conic helix is defined as a three-dimensional curve that has the general form of a conic helix except that the helix angle, defined between a tangent to the curve at any point and the central axis of the curve, is not constant as with a regular helix, but varies.

In one form, said primary cutting edge helix angle decreases to approximately zero degrees adjacent said drill tip.

Preferably, each said primary cutting edge extends at least substantially tangentially from the corresponding said secondary edge, when viewed in a plane extending tangentially through said secondary edge at a junction between said primary cutting edge and said secondary edge and extending perpendicular to said central axis.

Typically each said tip face blends into said flute leading side wall of the trailing adjacent said flute.

Typically, each of said flutes extends to within 0.1 mm of said drill tip, more typically within 0.05 mm of said drill tip.

Typically, the transverse cross-sectional area of a web of said drill bit, defined between the bases of each of said flutes, decreases in size along said cutting end part toward said drill tip.

Typically, each said tip face comprises:
a leading face margin defining said primary cutting edge; and
a face relief extending from said face margin toward the trailing adjacent said flute, said face relief blending into said flute leading side wall of said trailing adjacent flute;
wherein, in any cross-sectional plane extending perpendicular to said central axis through said cutting end part, said face margin of each said tip face lies in a circle extending about said central axis and each said face relief lies entirely within said circle.

Typically, in substantially any cross-sectional plane extending perpendicular to said central axis through said body, said secondary edge of each said land is convexly curved.

Typically, in substantially any said cross-sectional plane, each said secondary edge has a radius of at least 0.20 mm.

In a preferred form, each said primary cutting edge has a primary cutting edge transition region adjoining the respective said secondary edge and, in substantially any cross-sectional plane extending perpendicular to said central axis through said primary cutting edge transition region, said primary cutting edge is convexly curved or chamfered.

In a preferred form, each said land comprises:
said secondary edge of said land;
a land margin adjoining said secondary edge of said land;
a land relief extending from said land margin toward the trailing adjacent said flute; and
a land transition region blending said land relief into said flute leading side wall of the trailing adjacent flute;
wherein, in any cross sectional plane extending perpendicular to said central axis through said body, said land margin of each said land lies on a circle extending about said central axis and each land relief and each said transition region lies entirely within said circle.

Typically, said drill bit has three said flutes.

Typically, said drill bit is an orthopaedic drill bit.

### Brief Description of the Drawings

A preferred embodiment of the present invention will now be described, by way of an example only, with reference to the accompanying drawings wherein:
Figure 1 is a perspective view of a drill bit;
Figure 2 is an enlarged perspective view of the cutting end part of the drill bit of Figure 1;
Figure 3 is a front elevation view of the drill bit of Figure 1;
Figures 3a through 3e are each cross-sectional views of the drill bit of Figure 1 taken at sections A-A to E-E of Figure 3 respectively;
Figure 4 is an end elevation view of the drill bit of Figure 1;

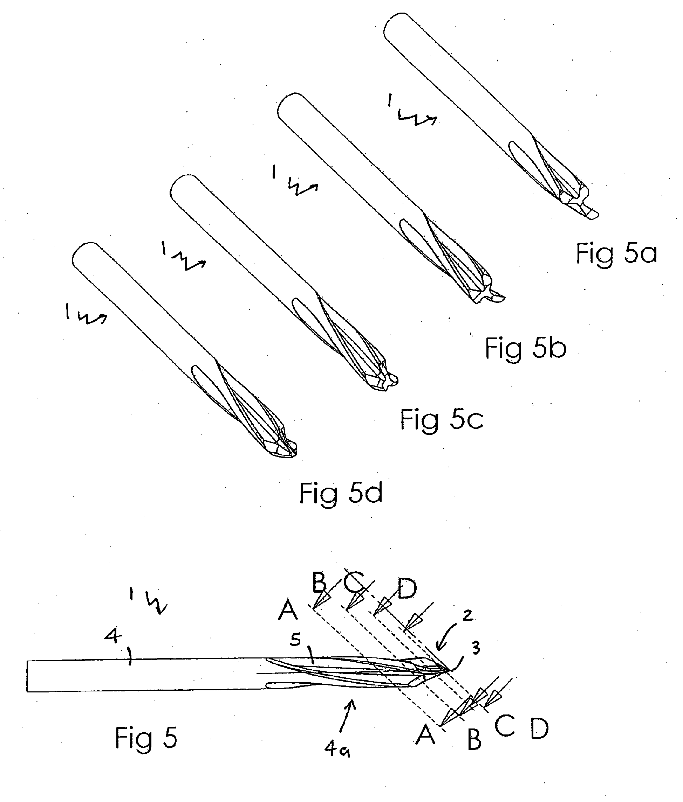

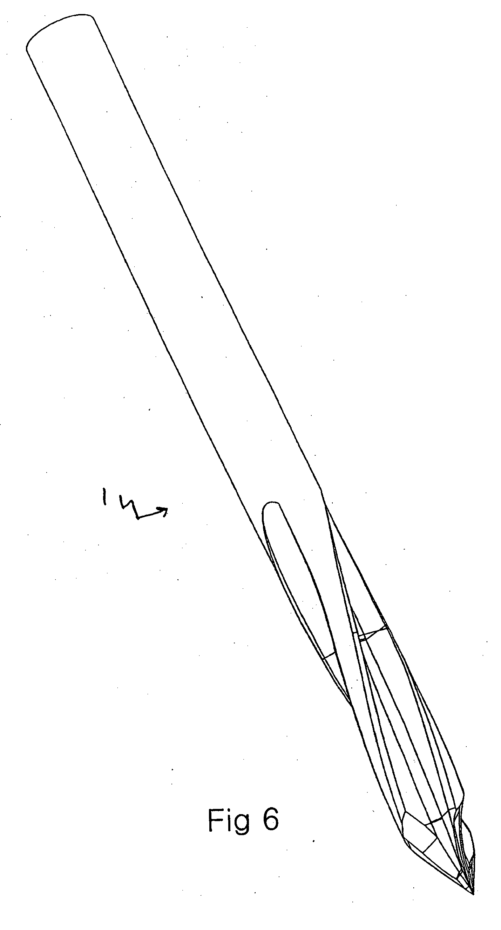

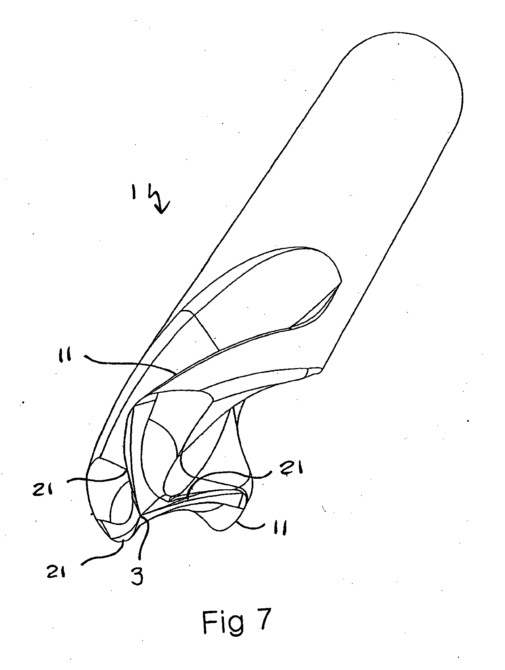

Figures 5 through 8 are each perspective/ fragmentary perspective views of the drill bit of Figure 1 as viewed from various angles.
Figure 9 is a graph depicting axial load vs time for the drill bit of Figure 1 drilling into bone material.

### Detailed Description of the Preferred Embodiments

Referring to the accompanying drawings, a drill bit 1 has a tapered cutting end part 2 terminating in a drill tip 3 at a front, operative end of the drill bit 1, with a shank 4 extending from an opposing rear end of the drill bit 1. The shank 4 is configured to be received within the chuck of a drill in the usual way, and will typically be cylindrical although it may be hexagonal in cross-section or any other suitable shape. A body 4a of the drill bit 1 extends between the cutting end part 2 and the shank 4. A plurality of flutes 5 are formed in the drill bit 1. In the embodiment depicted there are three flutes 5 that each generally helically extend along the body 4a from adjacent the shank 4 into the cutting end part 2. Each of the flutes 5 extends into the cutting end part 2 to adjacent the drill tip 3. Each of the flutes 5 comprises a flute body region 5b extending along the body 4a, and a flute end region 5a extending along the cutting end part 2 from the junction between the cutting end part 2 and body 4a to adjacent the drill tip 3.

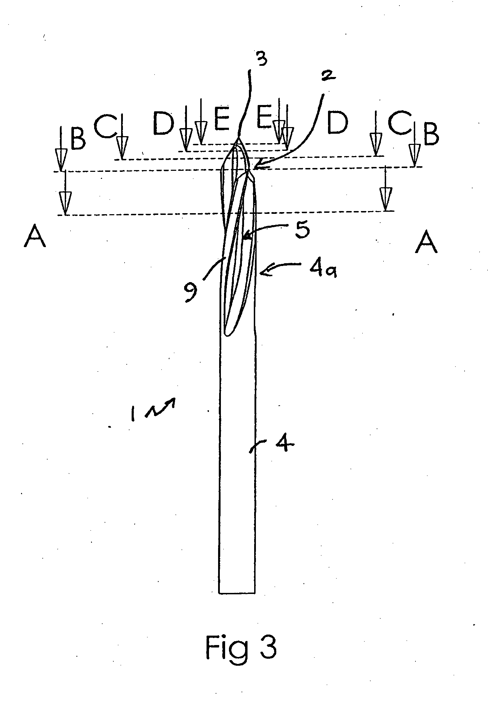

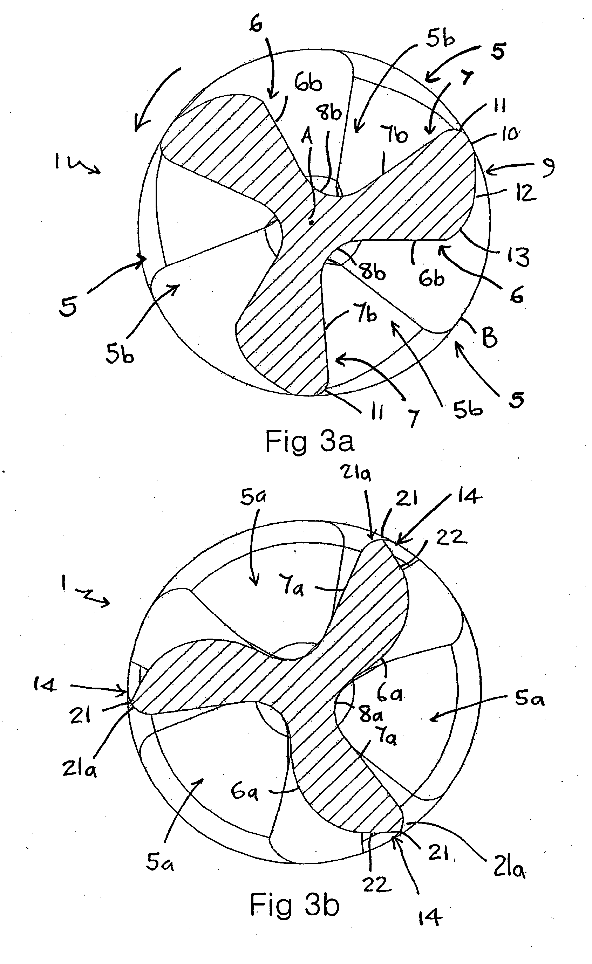

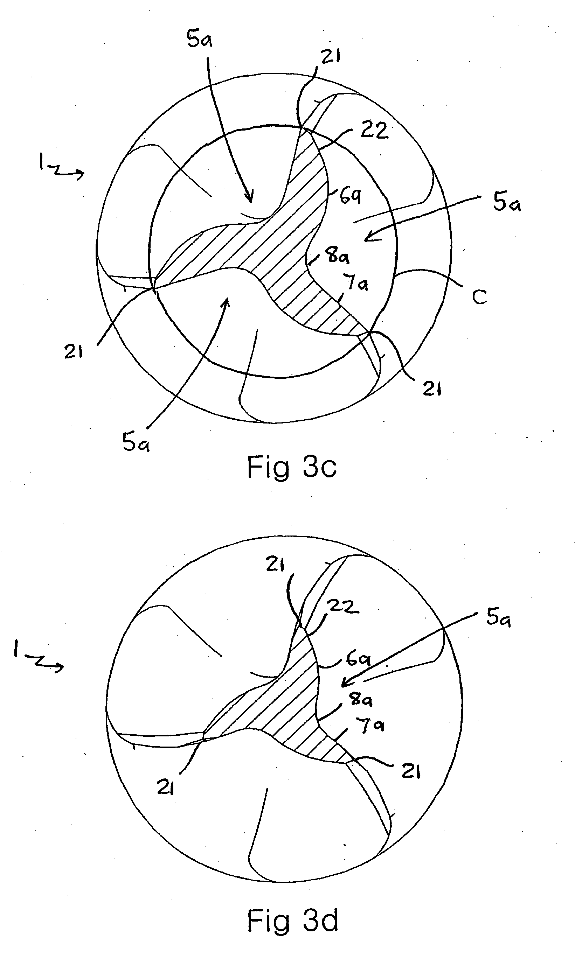

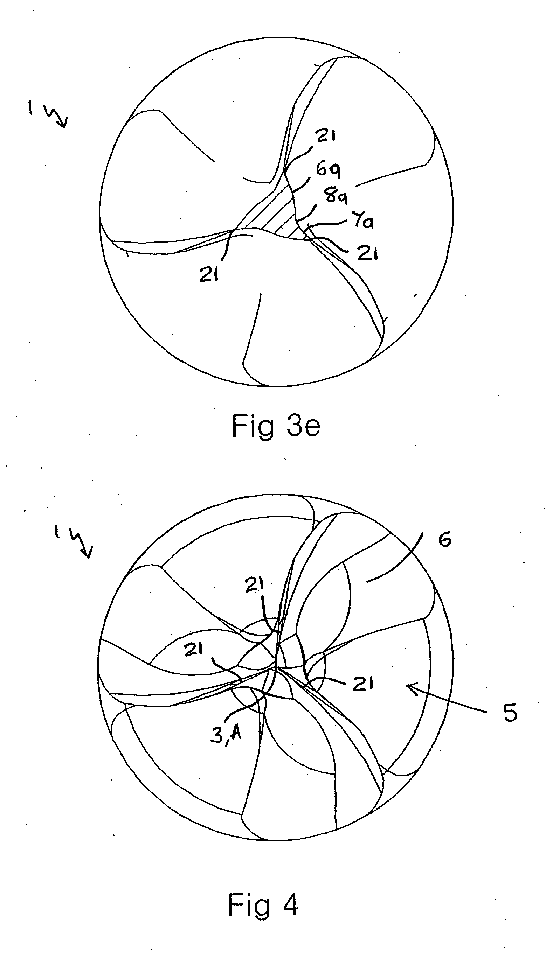

A land 9 is defined on the body 4a between each of the flutes 5. As best depicted in the cross-sectional view of Figure 3a, each land 9 has a land leading edge region defining a secondary edge 11 adjoining the adjacent flute trailing side wall 7 of the adjacent flute 5 directly leading the land 9. A land margin 10 is defined adjoining and trailing the secondary edge 11. Each land 9 also has a land relief 12 which extends from the land margin 10 towards the adjacent flute leading side wall 6 of the flute 5 directly trailing the land 9.

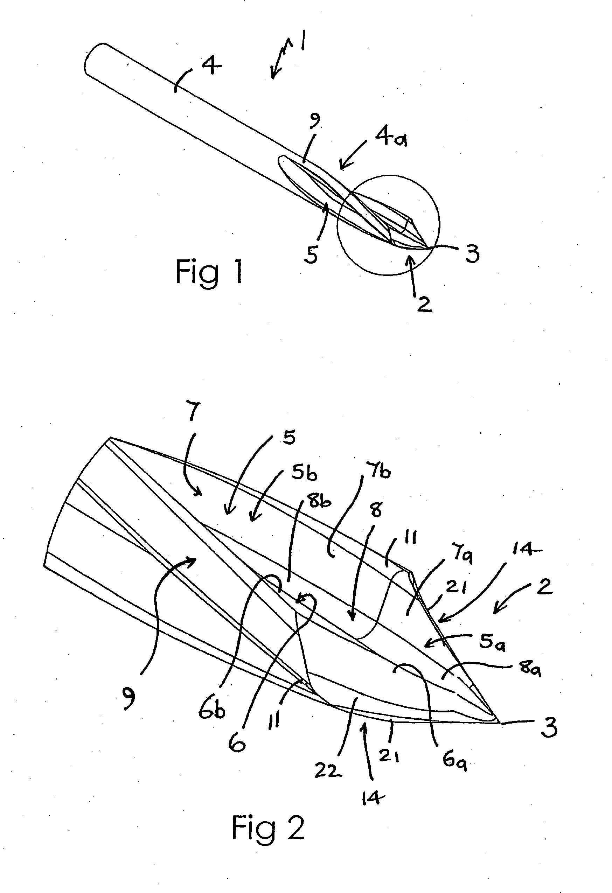

Referring specifically to Figure 2, the tapered cutting end part 2 of the drill bit 1 comprises three tip faces 14, one corresponding to each of the lands 9. Each tip face 14 extends from the corresponding land 9 to the drill tip 3 and effectively constitutes a tapered end of the corresponding land 9. The tip faces 14 define an included drill point angle, which is about 60° in the embodiment depicted, although the drill point angle may be altered as desired to suit the material to be drilled. Drill point angles of between 40° and 80° would be typical. The tip faces 14 are separated by the flutes 5 (in particular the flute end regions 5a) up to adjacent the drill tip 3, at the end of the flutes 5.

In the embodiment depicted, the drill bit 1 is configured to be rotated in a clockwise direction when viewed from the rear of the drill bit 1. Throughout this specification, various features of the drill bit will be referred to as "leading" or "trailing", with this terminology indicating features that lead or trail respectively as the drill bit rotates in the intended manner. As described above, each of the flutes 5 has a flute leading side wall 6 (which faces against the intended direction of rotation) and a flute trailing side wall 7 (which faces in the intended direction of rotation). The flute leading side wall 6 is joined to the flute trailing side wall 7 by way of a flute base 8 located therebetween. As best depicted in the cross-sectional views of Figures 3a through 3e, the flute leading side wall 6, flute base 8 and flute trailing side wall 7 effectively form a smooth continuous surface. The flute leading side wall 6, flute trailing side wall 7 and flute base 8 may be divided into end and body regions, in the same manner as each flute 5 has been divided into a flute end region 5a and flute body region 5b, and numbered accordingly. That is, the flute leading side wall 6 may be divided into a flute leading side wall end region 6a and flute leading side wall end region 6b, the flute trailing side wall 7 may be divided into a flute trailing side wall end region 7a and flute trailing side wall body region 7b and the flute base may be divided into a flute base end region 8a and flute base body region 8b.

The flute body regions 5b are each formed with a constant helix angle of about 13° in the embodiment depicted, although the helix angle may be adjusted as desired for different applications. Typical helix angles will be between 10° and 45°. The secondary edges 11 are formed with the same constant helix angle. The helix of each of the flutes 5 is configured such that the rear end of each flute 5 trails the front end as the drill bit 1 rotates in the intended direction. The flute base trailing regions 8b have a slight taper of about 1 degree with respect to the central axis A of the drill bit, reducing the depth of the flute trailing regions 5b towards the shank 4. The flute base end regions 8a have a larger taper with respect to the central axis A of the drill bit such that the area of the transverse cross-section of the web defined between the flute base end regions 8a reduces towards the drill tip 3 thereby allowing the flute end regions 5a to extend to adjacent the drill tip 3. The flutes 5 would otherwise terminate at a greater distance from the drill tip 3 as a result of the taper of the cutting end part 2. Typically, the flute end regions 5a extend to within 0.1 mm of the drill tip (equating to within about 0.02 times the diameter of a 4.5 mm diameter drill bit), or more typically within about 0.05 mm (0.01 times the drill bit diameter). In the particular embodiment depicted, the flute end regions 5a extend to within about 0.04 mm of the drill tip 3.

As best depicted in the various cross-sectional views of Figures 3b to 3e, each tip face 14 defines a primary cutting edge 21 with the flute trailing side wall end region 7a of the flute 5 that is directly leading the tip face 14.

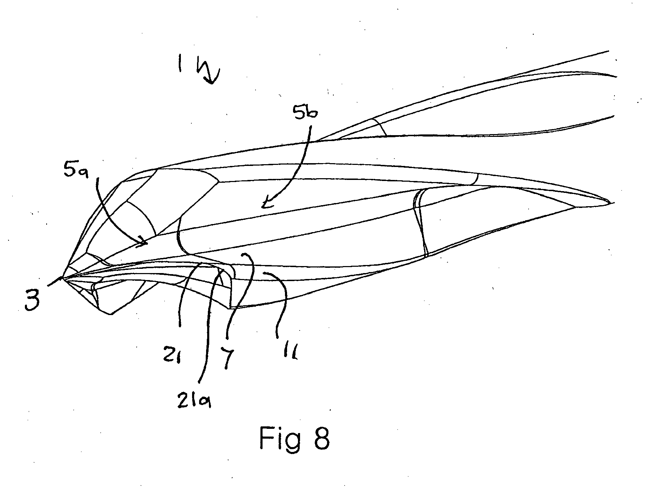

Each flute end region 5a is formed to provide a primary cutting edge 21 that extends from the corresponding secondary edge 11, at the intersection between the body 4a and cutting end part 2, in a variable conic helix type manner with a primary cutting edge helix angle that decreases from substantially equal to the secondary edge helix angle at the secondary edge 11 toward zero degrees as it approaches the drill tip 3. As noted above, in the context of the present specification, a variable conic helix is defined as a three-dimensional curve that has the general form of a conic helix except that the helix angle, defined between a tangent to the curve (here the primary cutting edge 21) and the central axis of the curve (here the central axis A), is not constant as with a regular helix, but varies. Accordingly, the primary cutting edge 21 gradually "straightens up" towards aligning with the central axis A as is perhaps most apparent in Figure 4 which shows the primary cutting edge helix angle of each primary cutting edge 21 decreasing to zero degrees adjacent to the drill tip 3 such that, when viewed from the drill tip end of the drill bit 1, the primary cutting edges 21 initially extend radially from adjacent the drill tip 3. As is perhaps best depicted in Figure 8, in the particular embodiment depicted, each primary cutting edge 21 extends at least substantially tangentially from the corresponding secondary edge 11 when viewed in a plane extending tangentially through the secondary edge 11 at the junction between the primary cutting edge 21 and the secondary cutting edge 11 and extending perpendicular to the central axis A. As a result, the flute end region 5a and flute body region 5b merge smoothly, particularly along the flute trailing side wall 7 and from the primary cutting edge 21 to the secondary edge 11.

As best depicted in Figures 3b and 8, each primary cutting edge 21 has a primary cutting edge transition region 21 a extending forward partway along the primary cutting edge 21 from the intersection with the secondary edge 11. In the primary cutting edge transition region 21a, the primary cutting edge 21 is convexly curved, with the radius of the primary cutting edge 21, taken in a cross-sectional plane extending perpendicular to the central axis A, increasing to blend the forward, sharp region of the primary cutting edge into the secondary edge 11 which, as discussed below, will typically be convexly curved. In the primary cutting edge transition region 21 a, the primary cutting edge 21 will typically have a radius, measured in the cross-sectional plane, that increases from zero at the forward end of the primary cutting edge transition region 21a to a radius equal to that of the secondary edge 11 (discussed further below), which will typically be at least 0.2 mm, typically between 0.2 mm and 0.5 mm, and here is about 0.3 mm.

As is apparent from Figure 3b, the convex curvature applied to the primary cutting edge 21 in the primary cutting edge transition region 21a might be construed as being applied to the adjacent radially outer region of the flute trailing side wall 7a of the leading adjacent flute 5, rather than being applied to the primary cutting edge 21 itself. With a configuration either applied or construed in such manner, the convex curvature of the adjoining radially outer region of the flute trailing side wall 7a results in the primary cutting edge 21 defining an increased included angle between the flute trailing side wall 21 and the adjoining tip face 14 equally results in a significantly less aggressive primary cutting edge 21 in the primary cutting edge transition region 21a. An equivalent effect could be achieved by, for example, providing a chamfer in the radially outer region of the flute trailing side wall 7a at the primary cutting edge 21 in the primary cutting edge transition region 21 a, rather than providing a convexly curved configuration. With such configurations, the included angle defined between the flute trailing side wall 7a and adjoining tip face 14 will typically increase towards the adjacent secondary edge 11, thereby reducing the aggressiveness of the primary cutting edge 21 as it approaches full diameter where it adjoins the secondary edge 11.

Typical prior art drills having a straight primary cutting edge and/or a primary cutting edge that ends well short of the drill tip. In such configurations, the cutting action provided toward the drill tip, being the first point of contact with the material to be drilled, is relatively unaggressive, with increased cutting power and aggressiveness being provided toward the rear end of the primary cutting edge toward the body. Toward the drill tip, the gradual reduction in depth of the flute generally results in a smaller included angle between the trailing side wall of the flute and the tip face, defining a less sharp cutting edge toward the tip, contributing to the low cutting aggressiveness and efficiency compared to the rear end of the primary cutting edge which generally provides a sharper primary cutting edge due to the steeper trailing side wall of the leading adjacent flute and greater speed of travel (owing to being located a greater distance from the centre of roatation). According to at least a preferred embodiment of the present invention, providing a primary cutting edge that extends in the general manner of a variable conic helix with a decreasing helix angle, a more aggressive and powerful cutting action may be achieved right up to toward the drill tip 3. Referring to Figure 4, this is understood to be a result, at least in part, of the fact that the rear portions of each primary cutting edge 21 rotationally lag the forward portions of the primary cutting edge 21 when the drill bit 21 is rotated in operation. Put another way, when viewed from the drill tip end of the drill tip 1 as in Figure 4, the rear portions of the primary cutting edge 21 can be seen to "fall behind" a radial line extending from the central axis A along the front portion of the primary cutting edge 21. This particular configuration can also be seen in Figure 4 to be akin to a ship's propeller. As a result of the configuration, at least of the preferred embodiment, more precise drilling of bone material in orthopaedic applications can be achieved more efficiently and more smoothly, reducing the possibility of damage to soft tissue by reducing the possibility of the radially outer portions of the primary cutting edge aggressively biting into soft tissue.

This is also enhanced by the configuration of the primary cutting edge 21 in the primary cutting edge transition region 21a, greatly reducing the aggressiveness of the primary cutting edge in the rear and radially outer portions of the primary cutting edge 11 towards the full diameter of the drill bit 1.

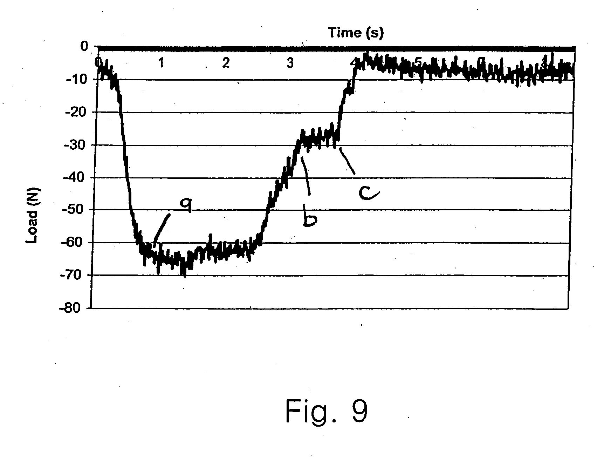

The particular effect of the configuration of the primary cutting edge 21 of the drill bit 1 of at least the preferred embodiment can be further explained with reference to Figure 9, which depicts the axial load against time required to be applied to a 4.5 mm prototype example of the drill bit 1 to achieve a constant axial feed rate of 5 mm/s through cortical bone material. The graph shows that the axial load applied roughly linearly increases as the drill tip 3 first engages the bone material until a peak load is rapidly achieved at point "a" (note that the load applied is depicted on a negative scale in Figure 9) following which the load gradually reduces, believed to be in part a result of the curved configuration of the primary cutting edge 21. The drill bit 1 further advances with reducing load until the primary cutting edge transition region 21a of the primary cutting edge 21 engages the surface of the material at point "b", just prior to the drill bit advancing through to the secondary cutting edge at full outer diameter of the drill bit. At this point, the gradual reduction in load is arrested or "braked" due to the greatly reduced cutting efficiency in the primary cutting edge region 21a of the primary cutting edge 21. The load then rapidly reduces to close to zero from point "c" where the drill bit breaks through the cortical bone. This is felt by the operator as a gradual reduction in pressure needed to be applied to the drill bit as it is advanced through the bone, with a short arrest in this load reduction providing tactile feedback to the operator to indicate that the drill is about to break through the cortical bone. The operator can use this feedback to reduce the axial load applied to the drill bit, arresting the rate of advancement of the drill bit as breakthrough is approached. Overshoot of the drill bit beyond the cortical bone into the soft tissue can thus be greatly reduced.

At the cutting end part 2, each tip face 14 blends into the flute leading side wall 6 of the flute 5 that is immediately trailing the tip face 14. In particular, in the arrangement depicted, each tip face 14 has a leading face margin 20 which defines the primary cutting edge 21 with the adjacent flute trailing side wall 7. In the embodiment depicted, the face margin 20 is particularly thin and is represented by a point in the cross-sections 3b through 3e. A face relief 22 extends from the face margin 20 towards the adjacent flute 5 immediately trailing the tip face 14 and smoothly blends into the flute leading side wall 6 of the trailing adjacent flute 5. Rather than blending smoothly into the flute leading side wall 7, it is also envisaged that the face relief 22 might blend into the flute leading side wall 6 by way of a series of chamfers when manufacturing capabilities are limited in their ability to grind a continuous smooth profile. As best depicted in Figure 3c, in any cross-sectional plane extending perpendicular to the central axis A through the cutting end part 2, the face margin 22 (here effectively defined by the primary cutting edge 21) of each tip face 14 lies in a circle C extending about the central axis A and each face relief 23 lies entirely within the circle C.

As can be seen in Figures 3b through 3e, the depth of each flute end region 5a reduces toward the drill tip 3 and the flute base leading region 8a gradually moves closer to the primary cutting edge 21 side of the flute profile, with the flute trailing side wall end region 6a remaining relatively straight so as to maintain a relatively sharp primary cutting edge 21 toward the drill tip 3.

The land leading edge region forming the secondary edge 11 of each land 9 may be convexly curved when viewed in any transverse cross-sectional plane extending perpendicular to the central axis A of the drill bit (such as the cross-sectional plane depicted in Figure 3a). The convex curvature may effectively be achieved by a series of discrete chamfers at the secondary edge 11. This may be contrasted to typical prior art drill bits that define a sharp cutting edge at the intersection between the leading edge of each land and the adjacent flute trailing side wall. As a result, the body 4a of the drill bit 1 may be provided with no secondary cutting edge, as is the case with typical known drill bits, leaving the entire cutting operation to the cutting end part 2, as will be described in further detail below. As a result, in the event that the operator moves the drill bit off-centre during the drilling process, there will be less tendency for the drill hole to be cut and widened by the misaligned body of the drill bit as compared to where sharp secondary cutting edges are provided on the body. A similar effect may be achieved by chamfering the land leading edge region. There is also a reduced possibility of damage to soft tissue in orthopaedic applications, reducing the possibility of the body of the drill bit biting into soft tissue and having soft tissue engaged and wrapped around the body. Providing a convexly curved or chamfered land leading edge region on each land also improves the smoothness of operation of the drill bit, reducing the aggressiveness of engagement of the drill bit, enabling provision of a smooth cutting process under decreased torque. These benefits may also be achieved without adversely affecting the structural integrity of the drill bit, maintaining a significant moment of inertia of the drill bit by not needing to reduce the amount of material in the body of the drill bit at full diameter to accommodate a greater angle between the flute trailing face and land to soften a secondary cutting edge.

For each land, the secondary edge 11 will typically have an average radius of at least 0.2 mm. In the particular embodiment depicted, the radius of the secondary edge 11 is approximately 0.3 mm. The radius of the secondary edge 11 may vary in any cross-sectional plane (that is, the land secondary edge 11 need not be formed as a constant radius arc). In any cross-sectional plane perpendicular to the central axis A extending through the body 4a, the average radius of each secondary edge 11 would typically be at least 0.04 times the overall diameter of the drill bit. It is also envisaged, however, that the secondary edge 11 of each land 9 may alternatively be of a standard sharp configuration.

In the particular arrangement depicted, a land transition region 13 blends the land relief 12 into the flute leading side wall 6, as best depicted in Figure 3a. This may be contrasted with a typical prior art drill bit wherein the land relief and flute leading side wall meet at a sharp edge (although it is envisaged that the curved land leading edge region described above may be used in conjunction with a conventional land relief/flute leading side wall). The transition region 13 will typically be curved so as to smoothly blend the land relief 12 into the flute leading side wall 6. The land transition region 13 will preferably have a radius, when measured in a cross-sectional plane perpendicular to the central axis A of the drill bit (such as the cross-sectional planes depicted in Figures 3a through 3e) of between 0.2 and 0.3 times the overall diameter of the drill bit 1. In the specific embodiment depicted, the drill bit 1 has an overall diameter of 4.5 mm, and the land transition region 13 has a radius of 1.15 mm. Rather than being curved, the land transition regions 13 could each be defined by one, or preferably two or more, chamfered surfaces.

The land margin 10 constitutes a part cylindrical portion of the land 9 which is not ground away from the cylindrical shaft from which the drill bit 1 is formed. The land margin 10 has a width (measured in a cross-sectional plane) of about 0.2 mm in the embodiment depicted, however, it is envisaged that the land margin 10 may have a minimal width, effectively defined by the intersection of the secondary edge 11 and the land relief 12. The land margins 10 each lie on a circle B extending about the central axis A and having a diameter equal to the overall diameter of the drill bit (being equal to the diameter of the shank 4 in the embodiment depicted). The secondary edge 11, land relief 12 and land transition region 13 of each land are ground away from the cylindrical shaft from which the drill bit 1 is formed. Accordingly, at any cross-sectional plane extending perpendicular to the central axis A through the lands 9, each secondary edge 11, land relief 12 and land transition region 13 lies entirely within the circle B as depicted in Figure 3a. The land relief 12 is convexly curved and is typically inclined with respect to the land margin 10 towards the central axis A, defining an edge between the land margin 10 and the land relief 12. Alternatively, the land relief 12 may gradually curve inwardly from the land margin 10 towards the central axis A without leaving any definite edge therebetween. In the embodiment depicted, the land relief 12 is inclined with respect to the land margin 10, when measured in a cross-sectional plane, by about 11° degrees at the junction therebetween. As such, the land relief 12 provides a greater area between its surface and the circle B than typical prior art designs, which are generally part cylindrical with a diameter only slightly less than the overall diameter of the drill bit.

The land relief 12 provides a clearance between the bulk of the land 9 and the wall of the hole being drilled, thereby further reducing drill bit drag. As stated above, this clearance is generally greater than that provided with typical prior art designs, particularly towards the land transition region 13. This increased clearance, together with the additional clearance provided by the land transition region 13, provides an additional, secondary flow path for swarf to pass along the drill bit 1 as the flutes 5 begin to fill and increase pressure. The land transition region 13 also provides an opportunity for swarf material that is not immediately passed into the flutes 5, but has travelled into the land relief 12, to flow into the flutes 5. The land transition region 13 is believed to create a region of reduced pressure which actively draws swarf from the land relief 12 into the adjacent flute 5. Efficiency of the drill bit 1 is thus not affected by a build-up of swarf material caught within the land relief region 12.

Each of the flute body regions 5b and adjacent secondary edge 11, land transition region 13 and land relief 12 is formed by grinding the shaft from which the drill bit 1 is formed in a single grinding operation with a single shaped grinding wheel. The flute end regions 5a and adjacent face reliefs 22 will typically be formed in a subsequent grinding operation with a single shaped grinding wheel extending along the cutting end part 2 from a position aligned with the central axis A and located very close to the central axis A and then progressing along the cutting end part 2, moving away from the central axis A and pivoting so as to blend into the flute body region 5b with the primary cutting edge 21 extending tangentially from the secondary edge 11 at the helix angle.

The drill bit will typically be formed of stainless steel when configured for use as an orthopaedic drill bit, but other suitable high strength metallic materials may be utilised as desired to suit various applications.

## Claims

1. A drill bit (1) having a central axis (A) and comprising:
a tapered cutting end part (2) terminating in a drill tip (3) at one end of said drill bit (1);
a shank (4) extending from an opposing end of said drill bit (1);
a body (4a) extending between said cutting end part (2) and said shank (4);
a plurality of flutes (5) formed in said drill bit (1) and generally helically extending along said body (4a) into said cutting end part (2) to adjacent said drill tip (3), each said flute (5) having a flute leading side wall (6) and a flute trailing side wall (7);
a land (9) defined on said body (4a) between each of said flutes (5) and extending to said cutting end part (2), each said land (9) defining a secondary edge (11) with said flute trailing side wall (7) of the leading adjacent said flute (5), each said secondary edge (11) helically extending to said cutting end part (2) at a secondary edge helix angle;
a plurality of tip faces (14) defined on said cutting end part (2) and each extending from one of said lands (9) to said drill tip (3), said tip faces (14) being separated by said flutes (5) up to adjacent said drill tip (3), each said tip face (14) defining a primary cutting edge (21) with said flute trailing side wall (7) of the leading adjacent said flute (5);
**characterised in that** each said primary cutting edge (21) extends from one of said secondary edges (11) in a variable conic helix type manner with a primary cutting edge (21) helix angle that decreases from substantially equal to said secondary edge helix angle at said secondary edge (11) toward zero as it approaches said drill tip (3).

2. The drill bit (1) of claim 1, wherein said primary cutting edge (21) helix angle decreases to approximately zero degrees adjacent said drill tip (3).

3. The drill bit (1) of claim 1, wherein each said primary cutting edge (21) extends at least substantially tangentially from the corresponding said secondary edge (11), when viewed in a plane extending tangentially through said secondary edge (11) at a junction between said primary cutting edge (21) and said secondary edge (11) and extending perpendicular to said central axis (A).

4. The drill bit (1) of claim 1, wherein each said tip face (14) blends into said flute leading side wall (6) of the trailing adjacent said flute (5).

5. The drill bit (1) of claim 1, wherein each of said flutes (5) extends to within 0.1 mm of said drill tip (3).

6. The drill bit (1) of claim 1, wherein each of said flutes (5) extends to within 0.05 mm of said drill tip (3).

7. The drill tip (3) of claim 1, wherein the transverse cross-sectional area of a web of said drill bit (1), defined between the bases (8) of each of said flutes (5), decreases in size along said cutting end part (2) toward said drill tip (3).

8. The drill tip (3) of claim 1, wherein each said tip face (14) comprises:
a leading face margin (20) defining said primary cutting edge (21); and
a face relief (22) extending from said face margin (20) toward the trailing adjacent said flute (5), said face relief (22) blending into said flute leading side wall (6) of said trailing adjacent flute (5);
wherein, in any cross-sectional plane extending perpendicular to said central axis (A) through said cutting end part (2), said face margin (20) of each said tip face (14) lies in a circle extending about said central axis (A) and each said face relief (22) lies entirely within said circle.

9. The drill bit (1) of claim 1, wherein, in substantially any cross-sectional plane extending perpendicular to said central axis (A) through said body (4a), said secondary edge (11) of each said land (9) is convexly curved.

10. The drill bit (1) of claim 1, wherein, in substantially any said cross-sectional plane, each said secondary edge (11) has a radius of at least 0.20 mm.

11. The drill bit (1) of claim 1, wherein each said primary cutting edge (21) has a primary cutting edge transition region (21a) adjoining the respective said secondary edge (11) and, in substantially any cross-sectional plane extending perpendicular to said central axis (A) through said primary cutting edge transition region (21 a), said primary cutting edge (21) is convexly curved or chamfered.

12. The drill bit (1) of claim 1, wherein each said land (9) comprises:
said secondary edge (11) of said land (9);
a land margin (10) adjoining said secondary edge (11) of said land (9);
a land relief (12) extending from said land margin (10) toward the trailing adjacent said flute (5); and
a land transition region (13) blending said land relief (12) into said flute leading side wall (6) of the trailing adjacent flute (5);
wherein, in any cross sectional plane extending perpendicular to said central axis (A) through said body (4a), said land margin ((10) of each said land (9) lies on a circle extending about said central axis (A) and each land relief (12) and each said transition region (13) lies entirely within said circle.

13. The drill bit (1) of claim 1, wherein said drill bit (1) has three said flutes (5).

14. The drill bit (1) of claim 1, wherein said drill bit (1) is an orthopaedic drill bit (1).

## Patentansprüche

1. Bohrmeißel (1) mit einer Mittelachse (A) und umfassend:
ein sich verjüngendes Schneidendteil (2), das in einer Bohrspitze (3) endet, an einem Ende des Bohrmeißels (1);
einen Schaft (4), der von einem gegenüberliegenden Ende des Bohrmeißels (1) verläuft;
einen Körper (4a), der zwischen dem Schneidendteil (2) und dem Schaft (4) verläuft;
mehrere Spannuten (5), die im Bohrmeißel (1) ausgebildet sind und im Allgemeinen spiralförmig den Körper (4a) entlang in das Schneidendteil (2) in die nähere Umgebung der Bohrspitze (3) verlaufen, wobei jede Spannut (5) eine Spannutvorderseitenwand (6) und eine Spannutrückseitenwand (7) aufweist;
eine Fase (9), die am Körper (4a) zwischen jeder der Spannuten (5) definiert ist und zum Schneidendteil (2) verläuft, wobei jede Fase (9) eine sekundäre Kante (11) mit der Spannutrückseitenwand (7) der vorderen benachbarten der Spannut (5) definiert, wobei jede sekundäre Kante (11) in einem sekundären Kantensteigungswinkel spiralförmig zum Schneidendteil (2) verläuft;
mehrere Spitzenflächen (14), die am Schneidendteil (2) definiert sind und jede von einer der Fasen (9) zur Bohrspitze (3) verlaufen, wobei die Spitzenflächen (14) durch die Spannuten (5) bis zur Nachbarschaft der Bohrspitze (3) getrennt sind, wobei jede Spitzenfläche (14) eine primäre Schneidkante (21) mit der Spannutenrückseitenwand (7) der vorderen benachbarten der Spannut (5);
**dadurch gekennzeichnet, dass** jede primäre Schneidkante (21) von einer der sekundären Kanten (11) in der Art einer variablen konischen Spiralform mit einem Steigungswinkel der primären Schneidkante (21) verläuft, der von ungefähr gleich dem Steigungswinkel der zweiten Kante an der zweiten Kante (11) nach Null hin abnimmt, wenn sie sich der Bohrspitze (3) annähert.

2. Bohrmeißel (1) nach Anspruch 1, wobei der Steigungswinkel der primären Schneidkante (21) der Bohrspitze (3) benachbart auf ungefähr null Grad abnimmt.

3. Bohrmeißel (1) nach Anspruch 1, wobei jede primäre Schneidkante (21) zumindest im Wesentlichen tangential von der entsprechenden sekundären Schneidkante (11) verläuft, bei Betrachtung in einer Ebene, die tangential durch die sekundäre Kante (11) an einer Verbindungsstelle zwischen der primären Schneidkante (21) und der sekundären Schneidkante (11) verläuft und senkrecht zur Mittelachse (A) verläuft.

4. Bohrmeißel (1) nach Anspruch 1, wobei jede Spitzenfläche (14) in die Spannutvorderseitenwand (6) der hinteren benachbarten Spannut (5) übergeht.

5. Bohrmeißel (1) nach Anspruch 1, wobei jede der Spannuten (5) bis zu innerhalb von 0,1 mm der Bohrspitze (3) verläuft.

6. Bohrmeißel (1) nach Anspruch 1, wobei jede der Spannuten (5) bis zu innerhalb von 0,05 mm der Bohrspitze (3) verläuft.

7. Bohrspitze (3) nach Anspruch 1, wobei die transversale Querschnittsfläche eines Stegs des Bohrmeißels (1), der zwischen den Basen (8) von jeder der Spannuten (5) definiert ist, in der Größe entlang des Schneidendteils (2) zur Bohrspitze (3) hin abnimmt.

8. Bohrspitze (3) nach Anspruch 1, wobei jede Spitzenfläche (14) Folgendes umfasst:
einen vorderen Flächenrand (20), der die primäre Schneidkante (21) definiert; und
ein Flächenrelief (22), das vom vorderen Flächenrand (20) zur hinteren benachbarten Spannut (5) verläuft, wobei das Flächenrelief (22) in die Spannutvorderseitenwand (6) der hinteren benachbarten Spannut (5) übergeht;
wobei in jeglicher Querschnittsebene, die senkrecht zur Mittelachse (A) durch das Schneidendteil (2) verläuft, der Flächenrand (20) jeder Spitzenfläche (14) in einem Kreis liegt, der um die Mittelachse (A) verläuft, und jedes Flächenrelief (22) vollständig innerhalb des Kreises liegt.

9. Bohrmeißel (1) nach Anspruch 1, wobei in im Wesentlichen jeglicher Querschnittsebene, die senkrecht zur Mittelachse (A) durch den Körper (4a) verläuft, die sekundäre Kante (11) jeder Fase (9) konvex gekrümmt ist.

10. Bohrmeißel (1) nach Anspruch 1, wobei in im Wesentlichen jeder Querschnittsebene jede sekundäre Kante (11) einen Radius von zumindest 0,20 mm aufweist.

11. Bohrmeißel (1) nach Anspruch 1, wobei jede primäre Schneidkante (21) einen primären Schneidkantenübergangsbereich (21 a) aufweist, der an die jeweilige sekundäre Kante (11) angrenzt, und wobei in im Wesentlichen jeder Querschnittsebene, die senkrecht zur Mittelachse (A) durch den primären Schneidkantenübergangsbereich (21 a) verläuft, die primäre Schneidkante (21) konvex gekrümmt oder abgeschrägt ist.

12. Bohrmeißel (1) nach Anspruch 1, wobei jede Fase (9) Folgendes umfasst:
die sekundäre Kante (11) der Fase (9);
einen Fasenrand (10), der an die sekundäre Kante (11) der Fase (9) angrenzt;
ein Fasenrelief (12), das vom Fasenrand (10) zur hinteren benachbarten Spannut (5) hin verläuft; und
einen Fasenübergangsbereich (13), der das Fasenrelief (12) in die Spannutvorderseitenwand (6) der hinteren benachbarten Spannut (5) übergehen lässt;
wobei in jeglicher Querschnittsebene, die senkrecht zur Mittelachse (A) durch den Körper (4a) verläuft, der Fasenrand (10) jeder Fase (9) in einem Kreis liegt, der um die Mittelachse (A) verläuft, und jedes Fasenrelief (12) und jeder Übergangsbereich (13) vollständig innerhalb des Kreises liegt.

13. Bohrmeißel (1) nach Anspruch 1, wobei der Bohrmeißel (1) drei Spannuten (5) aufweist.

14. Bohrmeißel (1) nach Anspruch 1, wobei der Bohrmeißel (1) ein orthopädischer Bohrmeißel (1) ist.

## Revendications

1. Trépan (1) présentant un axe central (A) et comprenant :
une partie d'extrémité de coupe conique (2) terminant en une pointe de mèche (3) à une extrémité dudit trépan (1) ;
une tige (4) s'étendant d'une extrémité opposée du trépan (1) ;
un corps (4a) s'étendant entre la partie d'extrémité de coupe (2) et la tige (4) ;
une pluralité de goujures (5) formées dans le trépan (1) s'étendant généralement en hélice le long du corps (4a) dans la partie d'extrémité de coupe conique (2) vers la pointe de mèche adjacente (3), chaque goujure (5) présentant une paroi latérale avant de goujure (6) et une paroi latérale arrière de goujure (7) ;
un chanfrein (9) défini sur le corps (4a) entre chacune des goujures (5) et s'étendant vers la partie d'extrémité de coupe (2), chaque chanfrein (9) définissant un bord secondaire (11) avec la paroi latérale arrière de goujure (7) de la goujure (5) avant adjacente, chaque bord secondaire (11) s'étendant en hélice vers la partie d'extrémité de coupe conique (2) à un angle d'hélice de bord secondaire ;
une pluralité de faces de pointe (14) définies sur la partie d'extrémité de coupe (2) et s'étendant chacune de l'un des chanfreins (9) vers la pointe de mèche (3), les faces de pointe (14) étant séparées par les goujures (5) jusqu'à la pointe de mèche adjacente (3), chaque face de pointe (14) définissant un bord de coupe principal (21) avec la paroi latérale arrière de goujure (7) de la goujure (5) avant adjacente ;
**caractérisé en ce que** chaque bord de coupe principal (21) s'étend de l'un des bords secondaires (11) d'une façon hélicoïdale conique variable avec un angle hélicoïdal du bord de coupe principal (21) qui diminue d'essentiellement égal à l'angle hélicoïdal du bord secondaire au dit bord secondaire (11) vers zéro lorsqu'il approche de la pointe de mèche (3).

2. Trépan (1) selon la revendication 1, dans lequel l'angle hélicoïdal du bord de coupe principal (21) se réduit à approximativement zéro degrés lorsqu'il est adjacent à la pointe de mèche (3).

3. Trépan (1) selon la revendication 1, dans lequel chaque bord de coupe principal (21) s'étend au moins essentiellement tangentiellement du bord secondaire (11) correspondant, lorsque vu dans un plan s'étendant tangentiellement à travers le bord secondaire (11) à une jonction entre le bord de coupe principal (21) et le bord secondaire (11) et s'étendant perpendiculairement à l'axe central (A).

4. Trépan (1) selon la revendication 1, dans lequel chaque face de pointe (14) se fond dans la paroi latérale avant de goujure (6) de la goujure (5) arrière adjacente.

5. Trépan (1) selon la revendication 1, dans lequel chacune des goujures (5) s'étend dans les limites de 0,1 mm de la pointe de mèche (3).

6. Trépan (1) selon la revendication 1, dans lequel chacune des goujures (5) s'étend dans les limites de 0,05 mm de la pointe de mèche (3).

7. Pointe de mèche (3) selon la revendication 1, dans lequel la zone de section transversale d'une âme du trépan (1), définie entre les bases (8) de chacune des goujures (5), diminue en taille le long de la partie d'extrémité de coupe (2) en direction de la pointe de mèche (3).

8. Pointe de mèche (3) selon la revendication 1, dans lequel la face de pointe (14) comprend :
un listel de face avant (20) définissant le bord de coupe principal (21) ; et
une dépouille de face (22) s'étendant du listel de face (20) en direction de la goujure (5) arrière adjacente, la dépouille de face (22) se fondant dans la paroi latérale avant de goujure (6) de la goujure (5) arrière adjacente ;
sachant que dans n'importe quel plan transversal s'étendant perpendiculairement à l'axe central (A) à travers la partie d'extrémité de coupe (2), le listel de face (20) de chaque face de pointe (14) repose dans un cercle s'étendant autour de l'axe central (A) et chaque dépouille de face (22) repose entièrement à l'intérieur du cercle.

9. Trépan (1) selon la revendication 1, dans lequel, dans essentiellement n'importe quel plan transversal s'étendant perpendiculairement à l'axe central (A) à travers le corps (4a), le bord secondaire (11) de chaque chanfrein (9) est incurvé de façon convexe.

10. Trépan (1) selon la revendication 1, dans lequel, dans essentiellement n'importe quel plan transversal, chaque bord secondaire (11) présente un rayon d'au moins 0,20 mm.

11. Trépan (1) selon la revendication 1, dans lequel chaque bord de coupe principal (21) présente une région de transition de bord de coupe principal (21 a) contiguë au bord secondaire (11) respectif et, dans essentiellement n'importe quel plan transversal s'étendant perpendiculairement à l'axe central (A) à travers la région de transition de bord de coupe principal (21 a), le bord de coupe principal (21) est incurvé de façon convexe ou chanfreiné.

12. Trépan (1) selon la revendication 1, dans lequel chaque chanfrein (9) comprend :
le bord secondaire (11) de chaque chanfrein (9) ;
un listel de chanfrein (10) contiguë au bord secondaire (11) du chanfrein (9) ;
une dépouille de chanfrein (12) s'étendant du listel de chanfrein (10) en direction de la goujure (5) arrière adjacente ; et
une région de transition de chanfrein (13) fondant la dépouille de chanfrein (12) dans la paroi latérale avant de goujure (6) de la goujure (5) arrière adjacente ;
sachant que dans n'importe quel plan transversal s'étendant perpendiculairement à l'axe central (A) à travers le corps (4a), le listel de chanfrein (10) de chaque chanfrein (9) repose sur un cercle s'étendant autour de l'axe central (A) et chaque dépouille de chanfrein (12) et chaque région de transition (13) repose entièrement à l'intérieur du cercle.

13. Trépan (1) selon la revendication 1, dans lequel le trépan (1) présente trois goujures (5).

14. Trépan (1) selon la revendication 1, dans lequel le trépan (1) est un trépan (1) orthopédique.
